# EUROPEAN PATENT APPLICATION

(11) **EP 4 187 449 A1**
(43) Date of publication of application: **31.05.2023**
(21) Application number: 22208041.8
(22) Date of filing: 17.11.2022
(51) Int. Cl.: G06N 5/022, G06N 20/00, G16H 20/10, G16H 70/40

(54) **INFORMATION PROCESSING PROGRAM, INFORMATION PROCESSING APPARATUS, AND INFORMATION PROCESSING METHOD**

(30) Priority: 24.11.2021 JP 2021189955
(71) Applicant: FUJITSU LIMITED, Kawasaki-shi, Kanagawa 211-8588 (JP)
(72) Inventor: UKAI, Takanori, Kawasaki-shi, Kanagawa, 211-8588 (JP)
(74) Representative: Hoffmann Eitle

(57) **Abstract**

An information processing program that causes a computer to execute a process that includes acquiring a graph dataset that includes graphs each having a subject, a predicate, and an object from a knowledge graph; generating a negative example graph dataset that includes one or more negative example graphs, a predicate of each negative example graph being same as a predicate of positive example graphs, an object of the negative example graph being different from an object of the positive example graphs, the negative example graph being excluded from the negative example graph dataset when the object of the negative example graph is different from each object to which a predicate of any other of the positive example graphs is linked; and training for embedding in the knowledge graph by using the positive example graph dataset and the negative example graph dataset.

## Description

### FIELD

Embodiments discussed herein are related to an information processing program and the like.

### BACKGROUND

For use of drugs, it is important to predict an unexpected action (side effect) in advance. Pharmaceutical manufacturers and distributors, those involved in pharmaceuticals, and the like are obliged to report to the Minister of Health, Labor and Welfare pursuant to the provisions of Article 68-10 of Pharmaceutical and Medical Device Act when they become aware of cases suspected to be due to side effects.

Pharmaceuticals and Medical Devices Agency (PMDA) discloses reports of domestic side effects reported from the manufacturers and distributors in a line list format and a comma-separated values (CSV) file format. Analysis and prediction are performed based on the data of these reports. For example, when a user inputs attributes, a disease, and an administered drug of a patient to an information processing apparatus, the information processing apparatus outputs a determination as to whether or not the patient has a possibility of a vein occlusion occurring as a side effect.

There has been disclosed a technique for providing information close to a user's search intention from knowledge graphs in consideration of the context of contents in searching.

There has been also disclosed a technique for embedding relational information composed of three data pieces (triples) in a vector space of a knowledge graph in order to predict links in the knowledge graph. An example of the technique for embedding in a vector space of a knowledge graph is TransE.

The technique for embedding in a vector space of a knowledge graph is a method for providing a vector representation for approximating a relationship among a subject, a property, and an object in the knowledge graph by an inner product similarity or the like. The knowledge graph embedding is considered as a promising technique for compensating for missing portions in knowledge graphs by prediction.

By using these techniques, it is possible to prepare knowledge graphs in which attributes, diseases, and administered drugs of patients are expressed, apply the technique for embedding in knowledge graphs to obtain vector data, and perform training by using the obtained vector data as compositions to predict a side effect.

Japanese Laid-open Patent Publication No. 2019-74843 is disclosed as related art.

Antonine Bordes et al., " Translating Embeddings for Modeling Multi-relational Data" is also disclosed as related art.

### SUMMARY

### [TECHNICAL PROBLEMS]

The training process used in the technique for embedding in a knowledge graph performs training by using positive example graph datasets and negative example graph datasets. For example, the training process performs the training by regarding linked data pieces in the knowledge graph as a positive example graph dataset, and unlinked data pieces in the knowledge graph as a negative example graph dataset. In the training process, a negative example graph dataset is generated by arbitrarily selection, and is used for training. For example, the training process arbitrarily selects obviously impossible graph datasets and uses the graph datasets for the training. For this reason, the training process using the technique for embedding in a knowledge graph has problems that training efficiency is low and a generated model achieves only low prediction accuracy.

According to an aspect of the embodiments, it is an object to improve training accuracy of the training process using the technique for embedding in a knowledge graph.

### [SOLUTION TO PROBLEM]

According to an aspect of the embodiments, an information processing program that causes at least one computer to execute a process, the process includes acquiring a graph dataset that includes one or more graphs each having a subject, a predicate, and an object from a knowledge graph; setting the acquired graph dataset as a positive example graph dataset that includes one or more positive example graphs; generating a negative example graph dataset that includes one or more negative example graphs each having a subject, a predicate, and an object, a predicate of each negative example graph being same as a predicate of one of the positive example graphs, an object of the negative example graph being different from an object of the one of the positive example graphs, the negative example graph being excluded from the negative example graph dataset when the object of the negative example graph is different from each object to which a predicate of any other of the positive example graphs is linked; and training for embedding in the knowledge graph by using the positive example graph dataset and the negative example graph dataset.

### [ADVANTAGEOUS EFFECTS OF INVENTION]

According to one embodiment, the training accuracy of a training process using the technique for embedding in a knowledge graph may be improved.

### BRIEF DESCRIPTION OF DRAWINGS

FIG. 1 is a functional block diagram illustrating a configuration of an information processing apparatus according to Embodiment 1;
FIG. 2 is a diagram illustrating an example of table data;
FIG. 3 is a diagram for explaining a conversion into graph datasets;
FIG. 4A is a diagram (1) for explaining generation of negative example datasets according to Embodiment 1;
FIG. 4B is a diagram (2) for explaining the generation of negative example datasets according to Embodiment 1;
FIG. 5A is a diagram (1) for explaining an example of a training process according to Embodiment 1;
FIG. 5B is a diagram (2) for explaining the example of the training process according to Embodiment 1;
FIG. 5C is a diagram (3) for explaining the example of the training process according to Embodiment 1;
FIG. 5D is a diagram (4) for explaining the example of the training process according to Embodiment 1;
FIG. 6 is a diagram presenting an example of a flowchart of the training process according to Embodiment 1;
FIG. 7 is a functional block diagram illustrating a configuration of an information processing apparatus according to Embodiment 2;
FIG. 8 is a diagram for explaining generation of negative example datasets according to Embodiment 2;
FIG. 9 is a diagram presenting an example of a flowchart of a training process according to Embodiment 2;
FIG. 10 is a functional block diagram illustrating a configuration of an information processing apparatus according to Embodiment 3;
FIG. 11A is a diagram (1) for explaining generation of negative example datasets according to Embodiment 3;
FIG. 11B is a diagram (2) for explaining the generation of the negative example datasets according to Embodiment 3;
FIG. 12 is a diagram presenting an example of a flowchart of a training process according to Embodiment 3;
FIG. 13 is a diagram illustrating an example of a computer that executes an information processing program; and
FIG. 14 is a diagram illustrating a reference example of generation of negative example datasets.

### DESCRIPTION OF EMBODIMENTS

Hereinafter, embodiments of an information processing apparatus, an information processing program, and an information processing method disclosed herein will be described in detail based on the drawings. The present disclosure is not limited by the embodiments.

First, description will be given of the technique for embedding relational information composed of multiple data pieces in a knowledge graph in order to perform link prediction in the knowledge graph (knowledge graph embedding technique). The knowledge graph embedding technique is a technique for embedding, in a vector space, a set of knowledge graphs each composed of three data pieces as a group of relational information, and converting vectors by a machine learning technique to obtain a predetermined data structure. The predetermined data structure refers to a data structure in which vectors V of respective h, r, and t where (h, r, t) (h: subject, r: predicate, and t: object) denote a group of relational information are such that Vₕ + Vᵣ is as equal as possible to Vₜ. Use of data structures thus machine-learned makes it possible to perform a calculation such as Vₕ + Vᵣ ≈ Vₜ, and thus predict t corresponding to Vₕ + Vᵣ. The use of the machine-learned data structures also makes it possible to predict h corresponding to Vₜ - Vᵣ and r corresponding to Vₜ - Vₕ.

As a group of relational information composed of three data pieces such as (h, r, t), data in a data format describing a relationship in which "a predicate of a subject is an object" is referred to as resource description framework (RDF) data. Although the RDF data is described which has a data structure including a group of three data pieces named a subject, a predicate, and an object, the subject may be referred to as a "subject", the predicate may be referred to as a "property", and the object may be referred to as an "object". Hereinafter, the subject will be referred to as a "subject", the predicate will be referred to as a "property", and the object will be referred to as an "object".

A training process used in the knowledge graph embedding technique performs training by using positive example graph datasets and negative example graph datasets. For example, the training process performs the training by treating data pieces linked in a knowledge graph as a positive example graph dataset, and data pieces unlinked in a knowledge graph as a negative example graph dataset. In the training process, a negative example graph dataset is generated by arbitrarily selection, and is used for training.

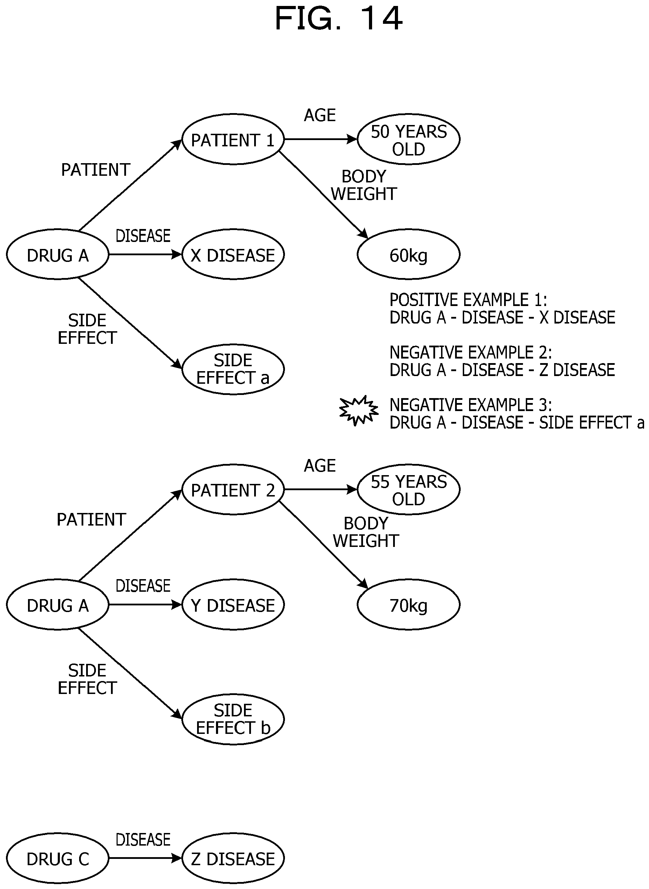

FIG. 14 is a diagram illustrating a reference example of generation of negative example datasets. In FIG. 14, multiple groups of relational information are present as a set of knowledge graphs. In the upper left in FIG. 14, there are graph datasets indicating relational information such as (drug A, patient, patient 1), (patient 1, age, 50), (patient 1, body weight, 60 kg), (drug A, disease, X disease), and (drug A, side effect, side effect a). In the middle left of FIG. 14, there are graph datasets indicating relational information such as (drug A, patient, patient 2), (patient 2, age, 55), (patient 2, body weight, 70 kg), (drug A, disease, Y disease), and (drug A, side effect, side effect b). In the lower left of FIG. 14, there is a graph dataset indicating relational information such as (drug C, disease, Z disease).

Under such a situation, in the training process, data pieces linked in a graph dataset is set as a positive example graph dataset. For example, since (drug A, disease, X disease) are linked, they are set as a positive example graph dataset (see a positive example 1). In contrast, (drug A, disease, Z disease) are set as a negative example graph dataset (see a negative example 2) by arbitrarily selecting "Z disease" because "Z disease" is unlinked to (drug A, disease). Even though "side effect a" is apparently unlinked to (drug A, disease), "side effect a" is arbitrarily selected and (drug A, disease, side effect a) are set as a negative example graph dataset (see a negative example 3). This "side effect a" is unlinked to "disease" represented as a property in any graph dataset. However, "side effect a" that is apparently unlinked to "disease" is arbitrarily selected and is set as a negative example graph dataset.

For this reason, the training process has problems that training efficiency is low and a generated training model achieves only low prediction accuracy.

To address this, the following embodiment will be described for an information processing apparatus that generates negative example graph datasets so as to improve training accuracy of a training process to be used in the knowledge graph embedding technique.

### [Embodiment 1]

### [Functional Configuration of Information Processing Apparatus]

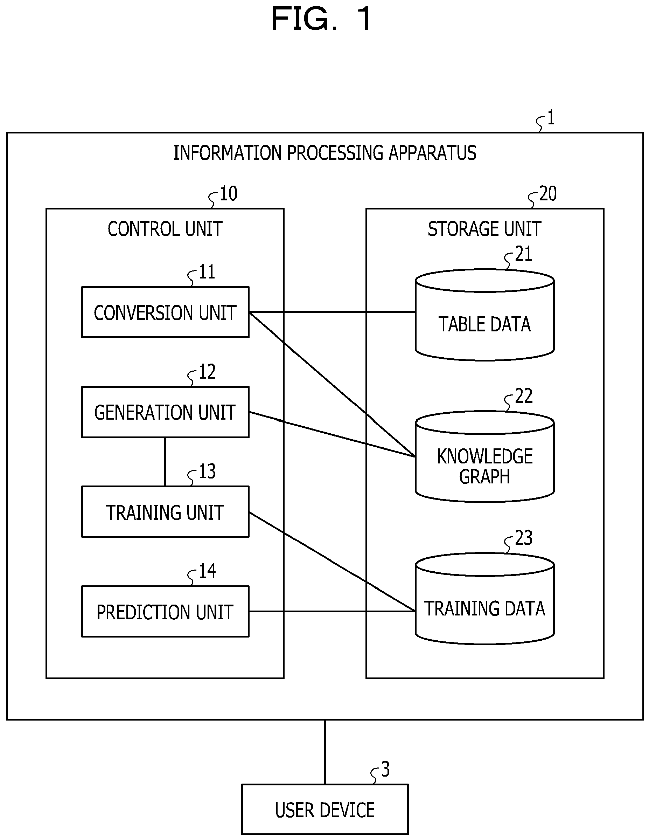

FIG. 1 is a functional block diagram illustrating a configuration of an information processing apparatus according to Embodiment 1. An information processing apparatus 1 illustrated in FIG. 1 acquires graph datasets each including a subject, a property, and an object from knowledge graphs, and sets the acquired graph datasets as positive example graph datasets. When generating negative example graph datasets each having the same property as the property linked to the object contained in a positive example graph dataset but having a different object, the information processing apparatus 1 uses only the objects linked to the property concerned.

The information processing apparatus 1 is coupled to a user device 3 and includes a control unit 10 and a storage unit 20. The control unit 10 includes a conversion unit 11, a generation unit 12, a training unit 13, and a prediction unit 14. The generation unit 12 is an example of an acquisition unit, a setting unit, and a generation unit. The training unit 13 is an example of a training unit.

The storage unit 20 stores various kinds of data. The storage unit 20 has table data 21, knowledge graphs 22, and training data 23.

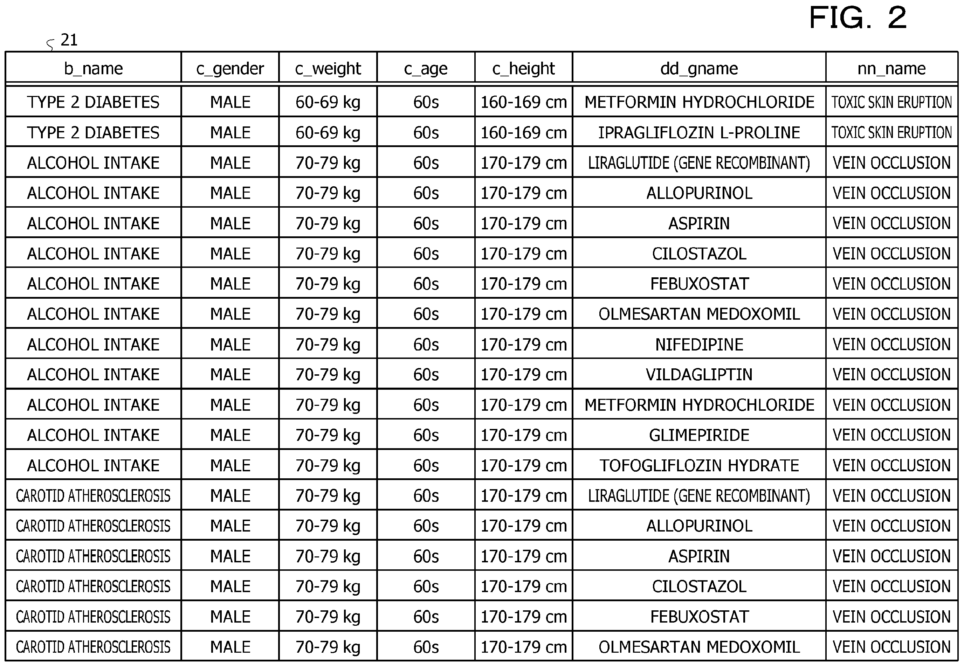

The table data 21 is a database (DB) that stores data to be converted into graph datasets. In the embodiment, the table data 21 is a DB that stores data requested for predicting a side effect in use of a drug. An example of the table data 21 will be described with reference to FIG. 2.

FIG. 2 is a diagram illustrating an example of table data. As illustrated in FIG. 2, the table data 21 is information in which items b_name, c_gender, c_wight, c_age, c_height, dd_gname, and nn_name are associated with each other. The item b_name is "disease". The item c_gender is "sex". The item c_wight is "body weight". The item c_age is "age". The item c_height is "height". The item dd_gname is "drug". The item nn_name is "side effect". Item names are set for each item.

As an example, in a case where an item name of b_name (disease) is "type 2 diabetes", "male" is stored as an item name of c_gender (sex), "60-69 kg" is stored as an item name of c_weight (body weight), "60s" is stored as an item name of c_age (age), and "160-169 cm" is stored as an item name of c_height (height). Additionally, "metformin hydrochloride" is stored as an item name of dd_gname (drug), and "toxic skin eruption" is stored as an item name of nn_name (side effect).

Returning to FIG. 1, the knowledge graphs 22 express relationships among various kinds of knowledge in a graph structure. Each knowledge graph 22 is information using relational information in which three data pieces are grouped. Each group of relational information includes three data pieces (triples) of a subject, a property, and an object. A specific example of the relational information is a resource description framework (RDF). A graph in which pieces of relational information are linked by arrows is referred to as a graph dataset.

As an example, ("drug A", "disease", "X disease") are stored as (subject, property, object) in the relational information. For example, this relational information has a relationship in which the "disease" of the "drug A" is the "X disease". In the relational information, ("drug A", "side effect", "side effect a") are stored as (subject, property, object). For example, this relational information has a relationship in which the "side effect "of the "drug A" is the "side effect a".

Returning to FIG. 1, the training data 23 is data resultant from training using graph datasets obtained by graphing the relational information. For example, the training data 23 contains a set of trained vectors for respective character strings contained in the subject, the property, and the object. The character string contains a word.

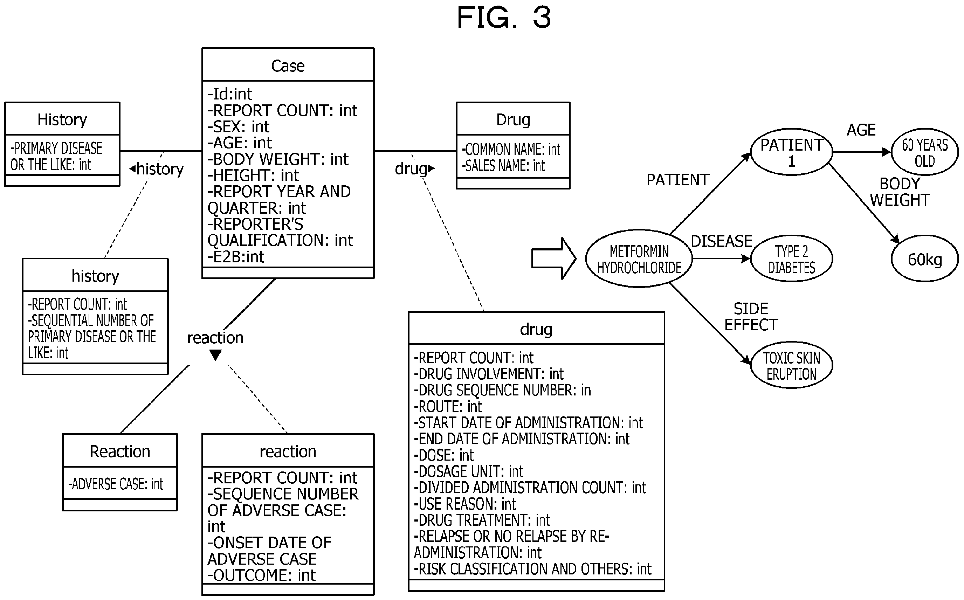

The conversion unit 11 converts the table data 21 into graph datasets. For example, the conversion unit 11 converts the table data 21 into graph datasets in accordance with an entity relationship (ER) diagram and generates the knowledge graphs 22 having the graph datasets. The conversion unit 11 concatenates each graph dataset with background knowledge. In the case of a drug, the background knowledge includes, for example, ingredients and the like of the drug. A conversion into graph datasets will be briefly described with reference to FIG. 3.

FIG. 3 is a diagram for explaining a conversion into graph datasets. An ER diagram is illustrated on the left side of FIG. 3. In the ER diagram, a Drug entity specifying a drug is linked (related) to a Case entity specifying attributes of a patient. The Drug entity specifying the drug is linked (related) to a History entity specifying a disease via the Case entity specifying the attributes of the patient. The Drug entity specifying the drug is linked (related) to a Reaction entity specifying a side effect via the Case entity specifying the attributes of the patient.

According to the ER diagram described above, the conversion unit 11 converts each line of the table data 21 into graph datasets. The knowledge graphs 22 having the graph datasets are illustrated on the right side of FIG. 3. In (subject, property, object), the subject and the object are represented by nodes, and the subject serves as a starting point whereas the object serves as an end point. The property is represented by a label beside an arrow.

For example, for the first line of the table data 21 illustrated in FIG. 2, the conversion unit 11 acquires the item name "metformin hydrochloride" of the dd_gname item and the item name "type 2 diabetes" of the b_name item in accordance with the relationship between the Drug entity and the History entity in the ER diagram. The conversion unit 11 converts the acquired item names into a graph dataset in which ("metformin hydrochloride", "disease", "type 2 diabetes") are set as the relational information. For the first line of the table data 21, the conversion unit 11 acquires the item name "metformin hydrochloride" of the dd_gname item and the item name "toxic skin eruption" of the nn_name item in accordance with the relationship between the Drug entity and the Reaction entity in the ER diagram. The conversion unit 11 converts the acquired item names into a graph dataset in which ("metformin hydrochloride", "side effect", "toxic skin eruption") are set as the relational information. The conversion unit 11 generates the knowledge graphs 22 having these graph datasets. In this embodiment, the b_name item in the table data 21 corresponds to "disease" represented as the property. The nn_name item in the table data 21 corresponds to the "side effect" represented as the property. Similarly, the c_wight item in the table data 21 corresponds to "body weight" represented as the property. The c_age item in the table data 21 corresponds to "age" represented as the property. The item name of each item corresponds to a character string represented as a subject or an object.

Returning to FIG. 1, the generation unit 12 generates positive and negative example graph datasets to be used for training. For example, the generation unit 12 acquires a graph dataset including a subject, a property, and an object from the knowledge graphs 22. For example, the generation unit 12 sets the acquired graph dataset as a positive example graph dataset. The generation unit 12 generates negative example graph datasets each having the same property as the property linked to the object contained in the positive example graph dataset but having a different object as follows. For example, the generation unit 12 narrows down negative example graph datasets to be generated and generates only negative example graph datasets each having the object existing in another positive example graph dataset. For example, the generation unit 12 sets, as training targets, only the item names contained in the item that is stored in the table data 21 and corresponds to the property contained in the positive example graph dataset. For example, the generation unit 12 excludes, from the training targets, the item names not contained in the item that is stored in the table data 21 and corresponds to the property contained in the positive example graph dataset. In a case where the item corresponding to the property contains the item names of multiple elements, the generation unit 12 generates negative example graph datasets by using the multiple elements as training targets. As an example, there is a case where the item corresponding to the property is "side effect" and the names of side effects and the names of diseases are contained as the item names of the "side effect" item. In this case, the generation unit 12 generates negative example graph datasets by using not only the side effects but also the diseases as the training targets.

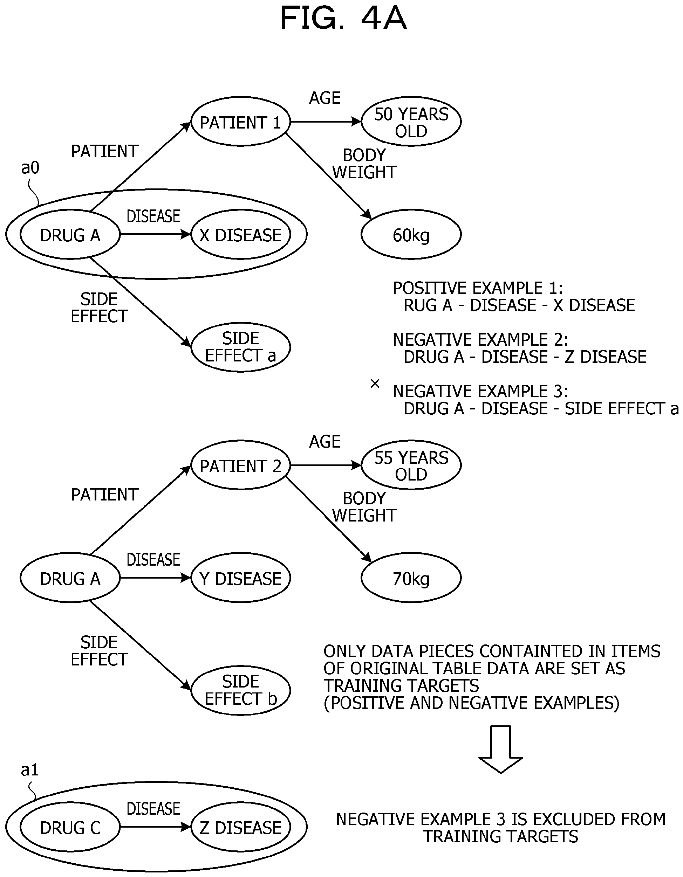

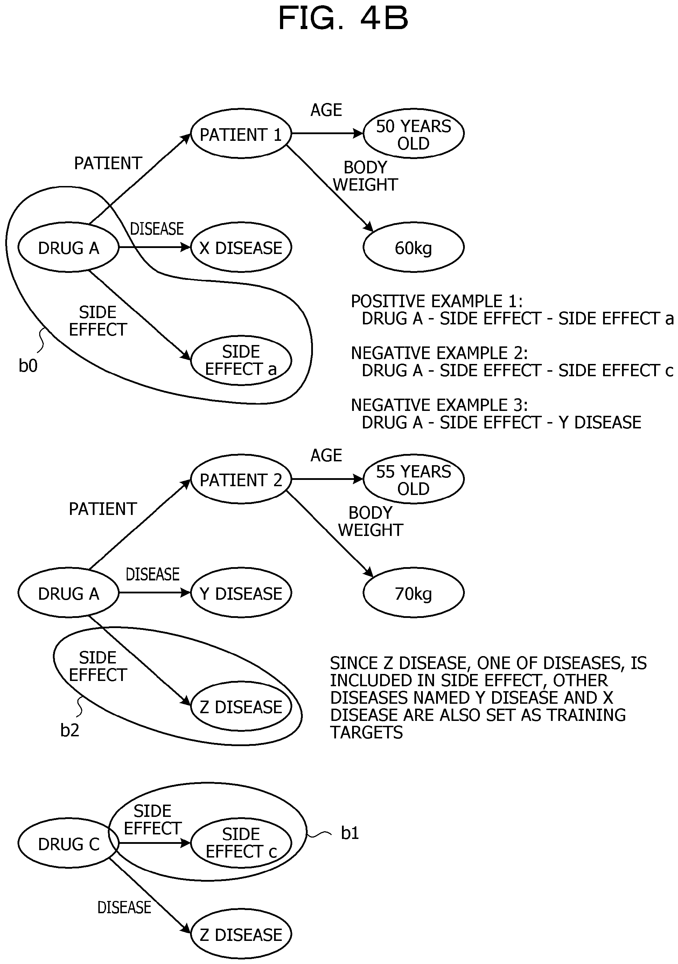

Generation of negative example datasets according to Embodiment 1 will be described with reference to FIGs. 4A and 4B. FIG. 4A is a diagram (1) for explaining generation of negative example datasets according to Embodiment 1. FIG. 4B is a diagram (2) for explaining the generation of negative example datasets according to Embodiment 1.

Knowledge graphs 22 are illustrated on the left side of FIG. 4A. The generation unit 12 acquires a graph dataset specifying a subject, a property, and an object from the knowledge graphs 22. For example, the generation unit 12 sets the acquired graph dataset as a positive example graph dataset. In this example, the generation unit 12 acquires a graph dataset a0 specifying ("drug A", "disease", "X disease") as relational information from the knowledge graphs 22. The generation unit 12 sets the acquired graph dataset a0 as a positive example graph dataset (see a positive example 1). The generation unit 12 may acquire a graph dataset specifying ("drug A", "disease", "Y disease") as relational information from the knowledge graphs 22, and set the acquired graph dataset as a positive example graph dataset instead of or in addition to the positive example 1.

The generation unit 12 generates a negative example graph dataset having the same property as the property contained in the positive example graph dataset but having a different object as follows. For example, the generation unit 12 acquires the other positive example graph datasets each having the same property as the property of the positive example graph dataset. The generation unit 12 narrows down negative example graph datasets to be generated and generates only negative example graph datasets each having the object contained in one of the other positive example graph datasets acquired. The generation unit 12 generates a negative example graph dataset having the same property as the property "disease" contained in the graph dataset a0 of the positive example 1 but having an object different from "X disease" as follows. For example, the generation unit 12 acquires another positive example graph dataset a1 having the property "disease". The generation unit 12 generates a negative example graph dataset having the object "Z disease" contained in the acquired graph dataset a1 (see a negative example 2).

For example, the generation unit 12 excludes, from targets for negative example graph datasets, item names not contained in the item that is stored in the table data 21 and corresponds to the property in the positive example graph dataset. The item name "side effect a" is contained in the item "side effect" corresponding to the property, but is not contained in the item "disease" corresponding to the property. Accordingly, the generation unit 12 excludes a graph dataset having the item name "side effect a" that is not contained in the item "disease" corresponding to the property from the targets for negative example graph datasets (see a negative example 3). For example, the generation unit 12 excludes "side effect a" that is apparently unlinked to "disease" from the targets for the negative example graph datasets.

Other knowledge graphs 22 are illustrated on the left side in FIG. 4B. When the item names of multiple elements are contained in the item corresponding to the property, the generation unit 12 generates negative example graph datasets by using the multiple elements as targets. In this example, the generation unit 12 acquires a graph dataset b0 specifying (drug A, side effect, side effect a) as relational information from the knowledge graphs 22. The generation unit 12 sets the acquired graph dataset b0 as a positive example graph dataset (see a positive example 1).

When the item names of multiple elements are contained in the item corresponding to the property, the generation unit 12 generates negative example graph datasets by using the multiple elements as targets. In FIG. 4B, the item "side effect" corresponding to the property contains the names of side effects and the name of a disease. In reference sign b0, the item "side effect" corresponding to the property contains "side effect a" as the name of a side effect. In reference sign b1, the item "side effect" corresponding to the property contains "side effect c" as the name of a side effect. In reference sign b2, the item "side effect" corresponding to the property contains "Z disease" as the name of a disease. For example, the item "side effect" corresponding to the property contains the item names of the multiple elements, which are the item names of "side effect" and the item name of "disease".

In this case, the generation unit 12 generates a negative example graph dataset having the object "side effect c" contained in the other positive example graph dataset b1 having the item "side effect" corresponding to the property (see a negative example 2).

In addition, the generation unit 12 uses "Y disease" and "X disease" as targets for negative example graph datasets because "Z disease" contained in "disease" exists in the item "side effect" corresponding to the property as illustrated in reference sign b2. For example, the generation unit 12 generates a negative example graph dataset in which the property is "side effect" and the object is "Y disease" contained in "disease" (see a negative example 3). The generation unit 12 may generate a negative example graph dataset in which the property is "side effect" and the object is "X disease" contained in "disease".

In this way, the generation unit 12 may generate graph datasets desired as negative example graph datasets by excluding data pieces apparently unlinked.

Returning to FIG. 1, the training unit 13 uses the positive example graph datasets and the negative example graph datasets to perform training for embedding in the knowledge graphs 22. For example, using a positive example graph dataset, the training unit 13 performs training such that the vector obtained by adding the vector of the property to the vector of the subject approaches the vector of the object. Using a negative example graph dataset, the training unit 13 performs training such that the vector obtained by adding the vector of the property to the vector of the subject becomes farther from the vector of the object. For example, the training unit 13 performs the training on the embedding of the vectors of the subject, the property, and the object based on the set of data pieces of triples (subject, property, object) in the graph dataset. The training unit 13 stores the training result in the training data 23. The training result stored in the training data 23 includes a set of trained vectors for the respective character strings contained in the subject, the property and the object.

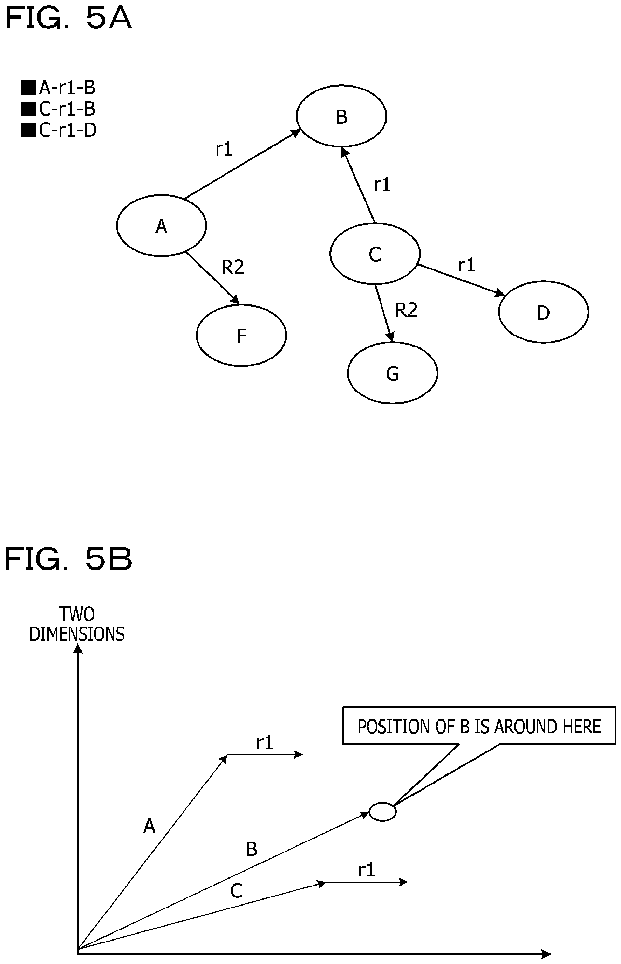

An example of a training process will be described with reference to FIGs. 5A to 5D. FIGs. 5A to 5D are diagrams for explaining an example of the training process according to Embodiment 1. Knowledge graphs 22 are illustrated in FIG. 5A. For example, (A, r1, B), (C, r1, B), and (C, r1, D) are represented as (subject, property, object) in the knowledge graphs 22. In this example, (A, r1, B), (C, r1, B), and (C, r1, D) are positive example graph datasets.

Each of items (subject, property, object) is already initialized in n dimensions. For convenience, description will be given by assuming two dimensions. The training unit 13 arranges the initialized vectors. The training unit 13 performs training such that the vector obtained by adding the vector of the property to the vector of the subject approaches the vector of the object. As illustrated in FIG. 5B, the training unit 13 causes the vector obtained by adding the vector of the property "r1" to the vector of the subject "A" and the vector of the object "B" to approach each other. For example, the training unit 13 performs mapping such that the "A" vector + the "r1" vector become closer to the "B" vector. The training unit 13 causes the vector obtained by adding the vector of the property "r1" to the vector of the subject "C" and the vector of the object "B" to approach each other. For example, the training unit 13 performs mapping such that the "C" vector + the "r1" vector become closer to the "B" vector.

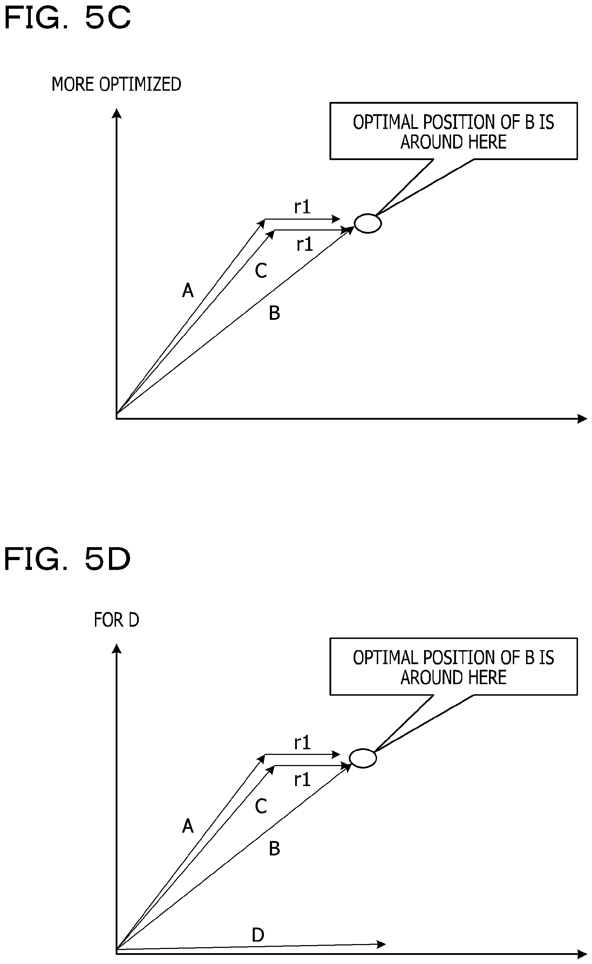

As illustrated in FIG. 5C, for further optimization, the training unit 13 causes the vector obtained by adding the vector of the property "r1" to the vector of the subject "A" and the vector of the object "B" to approach each other. The training unit 13 causes the vector obtained by adding the vector of the property "r1" to the vector of the subject "C" and the vector of the object "B" to approach each other. In this way, the position of the vector of "B" is optimized.

As illustrated in FIG. 5D, for the object "D", the training unit 13 causes the vector obtained by adding the vector of the property "r1" to the vector of the subject "C" and the vector of the object "D" to approach each other. For example, the training unit 13 maps the object "D" at a position close to the "C" vector + the "r1" vector and far from the "A" vector + the "r1" vector.

In this way, the training unit 13 iterates the above processing on each positive example graph dataset until its inter-vector distance is sufficiently reduced. The number of iterations is determined in advance as one of hyperparameters. As a result, the training unit 13 generates the vectors between which the distance is sufficiently reduced as a training result. The training unit 13 stores the training result in the training data 23. The training result is a set of the trained vectors. Although not illustrated, the training unit 13 may perform training on a negative example graph dataset such that the vector obtained by adding the vector of the property to the vector of the subject becomes farther from the vector of the object.

Returning to FIG. 1, the prediction unit 14 predicts a prediction target of the input relational information by using the trained vectors. For example, the prediction unit 14 uses a set of trained vectors stored in the training data 23 to predict a prediction target of the input relational information as follows. From the set of trained vectors, the prediction unit 14 acquires vectors corresponding to two character strings other than the prediction target of the input relational information. The prediction unit 14 selects a vector one by one from the set of trained vectors. The prediction unit 14 uses the vectors of the character strings other than the prediction target and each selected vector to obtain a vector by subtracting the vector of the object from the vector obtained by adding the vector of the property to the vector of the subject and searches the obtained vectors for a vector smaller than a predetermined score. The prediction unit 14 predicts a character string corresponding to the searched-out vector as the prediction target.

As an example, in a case of prediction of "what is the "side effect" of "drug D"?", (subject, property, object) are ("drug D", "side effect", t), and the object is a prediction target. In this case, the prediction unit 14 searches for the selected vector Vₜ such that the vector obtained by subtracting the selected vector Vₜ from the vector obtained by adding the vector Vᵣ of "side effect" to the vector Vₕ of "drug D" is smaller than the score. The prediction unit 14 predicts the character string t corresponding to the selected vector Vₜ, which is searched out, as the prediction target.

### [Flowchart of Training Process]

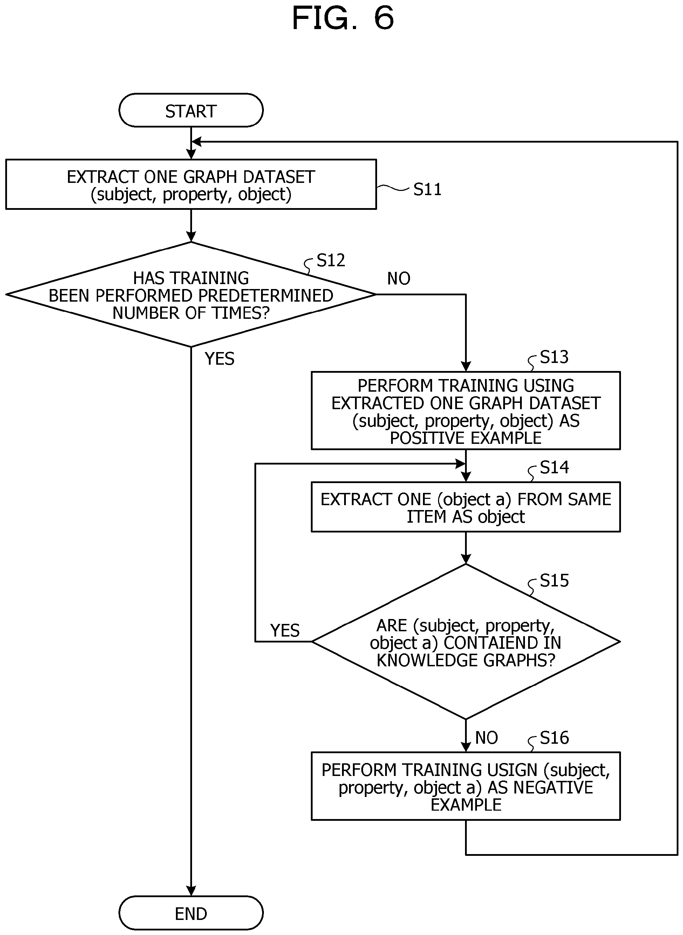

FIG. 6 illustrates an example of a flowchart of the training process according to Embodiment 1. The conversion unit 11 converts the table data 21 into the knowledge graphs 22.

As illustrated in FIG. 6, the training unit 13 extracts one graph dataset (subject, property, object) from the knowledge graphs 22 (step S11). The training unit 13 determines whether or not training has been performed a predetermined number of times (step S12). When determining that the training has been performed the predetermined number of times (step S12; Yes), the training unit 13 ends the training process.

On the other hand, when determining that the training has not been performed the predetermined number of times (step S12; No), the training unit 13 performs the training using the extracted one graph dataset (subject, property, object) as a positive example (step S13). For example, the training unit 13 uses the positive example graph dataset to perform training for embedding in the knowledge graphs 22.

The generation unit 12 extracts one object a from the same item as the object (step S14). For example, the generation unit 12 extracts another object contained in the same item as the object from the table data 21. For example, as negative example graph datasets each having the same property as the property linked to the object contained in the positive example graph dataset but having a different object, the generation unit 12 generates only negative example graph datasets each having an object present in another positive example graph dataset. As an example, in a case where the positive example graph dataset is (drug A, side effect, side effect a), the generation unit 12 generates only the negative example graph datasets having the different objects "side effect c" and "Z disease" contained in the item "side effect (nn_name)" which is the item in the table dataset 21 and is the same as the object "side effect a" (see FIG. 4B).

The generation unit 12 determines whether or not (subject, property, object a) are contained in the knowledge graphs 22 (step S15). When determining that (subject, property, object a) are contained in the knowledge graphs 22 (step S15; Yes), the generation unit 12 proceeds to step S14 to extract a next object.

On the other hand, when determining that (subject, property, object a) are not contained in the knowledge graphs 22 (step S15; No), the training unit 13 performs the training by using (subject, property, object a) as a negative example (step S16). For example, the training unit 13 uses the negative example graph dataset to perform the training for embedding in the knowledge graphs 22. After that, the training unit 13 proceeds to step S11 in order to extract a next graph dataset.

### [Effects of Embodiment 1]

According to Embodiment 1 described above, the information processing apparatus 1 acquires graph datasets each containing a subject, a property, and an object from the knowledge graphs 22 in the training for embedding in the knowledge graphs 22. The information processing apparatus 1 sets the acquired graph datasets as positive example graph datasets. When the information processing apparatus 1 generates negative example graph datasets each having the same property as the property linked to the object contained in one of the positive example graph datasets but having a different object, the information processing apparatus 1 narrows down negative example graph datasets to be generated and generates only negative example graph datasets each having another object linked to the concerned property existing in another positive example graph dataset. By using the positive example graph datasets and the negative example graph datasets, the information processing apparatus 1 performs the training for embedding in the knowledge graphs 22. In this way, by using only objects that exist in other positive example graph datasets for negative example graph datasets, the information processing apparatus 1 is capable of excluding an object that is apparently unlinked and stopping generation of unnecessary negative example graph datasets. As a result, the information processing apparatus 1 skips the training on unnecessary negative example graph datasets, so that it is possible to speed up convergence of the training and improve prediction accuracy.

### [Embodiment 2]

In Embodiment 1, when the information processing apparatus 1 generates a negative example graph dataset having the same property as the property linked to an object contained in a positive example graph dataset but having a different object, the information processing apparatus 1 generates the negative example graph dataset to be generated as follows. As described above, the information processing apparatus 1 generates only the negative example graph datasets each having the object existing in another positive example graph dataset. However, the information processing apparatus 1 is not limited to this. Instead, in a case where a class of every object is set in an ontology, the information processing apparatus 1 may generate only negative example graph datasets each having an object in the same class as the class of the object contained in a positive example graph dataset.

Embodiment 2 will be described for a case where the information processing apparatus 1 generates only negative example graph datasets each having an object in the same class as the class of the object contained in a positive example graph dataset when the class of every object is set in the ontology.

### [Functional Configuration of Information Processing Apparatus]

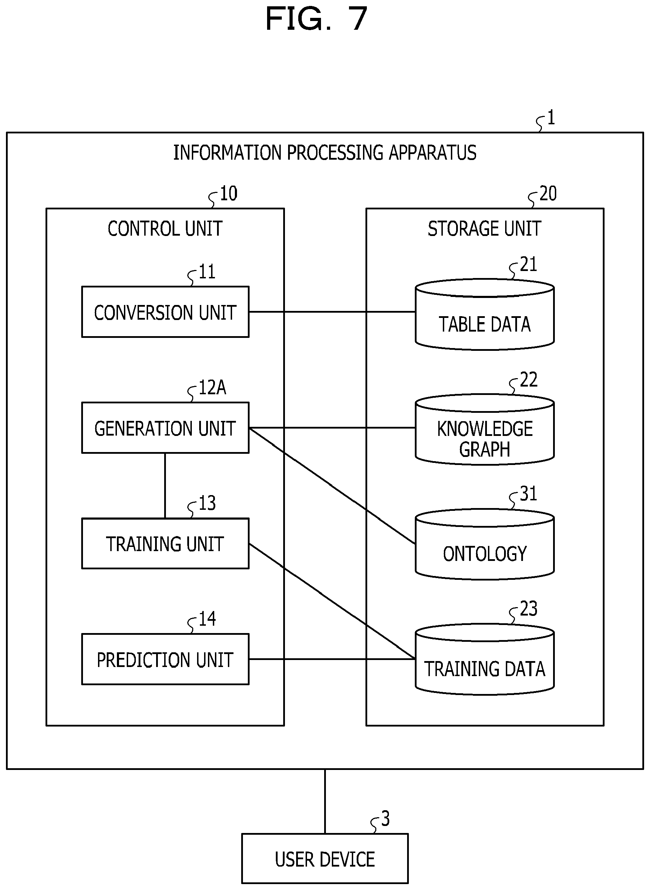

FIG. 7 is a functional block diagram illustrating a configuration of an information processing apparatus according to Embodiment 2. The same constituent elements as in the information processing apparatus 1 illustrated in FIG. 1 are assigned with the same reference sign, and the description of the identical constituent elements and operations thereof is omitted herein. Embodiment 2 is different from Embodiment 1 in that the generation unit 12 is replaced by a generation unit 12A. Embodiment 2 is different from Embodiment 1 in that an ontology 31 is added.

The ontology 31 provides data used in a certain domain for modeling of the certain domain. The ontology 31 is information on the certain domain using relational information in which three data pieces are grouped. Each group of relational information includes three data pieces (triples) of a subject, a property, and an object. A class (type) of every data piece is set in the ontology 31. As an example, "disease name" is set as a class of "X disease", "Y disease", and "Z disease". Then, "reaction" is set as a class of "side effect a" and "side effect c". These classes are defined by international standards.

The generation unit 12A generates positive and negative example graph datasets to be used for training. For example, the generation unit 12A acquires a graph dataset composed of a subject, a property, and an object from the knowledge graphs 22. The generation unit 12A sets the acquired graph dataset as a positive example graph dataset. By referring to the ontology 31, the generation unit 12A generates negative example graph datasets each having an object in the same class as the class of the object contained in the positive example graph dataset. For example, when objects in multiple classes may exist in the objects linked to the property, the generation unit 12A generates negative example graph datasets by using the objects in the multiple classes as training targets. As an example, in a case where an object "Y disease" in a "disease name" class and an object "side effect c" in a "reaction" class exist in a property "side effect", the generation unit 12A generates negative example graph datasets by using objects in the "disease name" class and the "reaction" class as training targets.

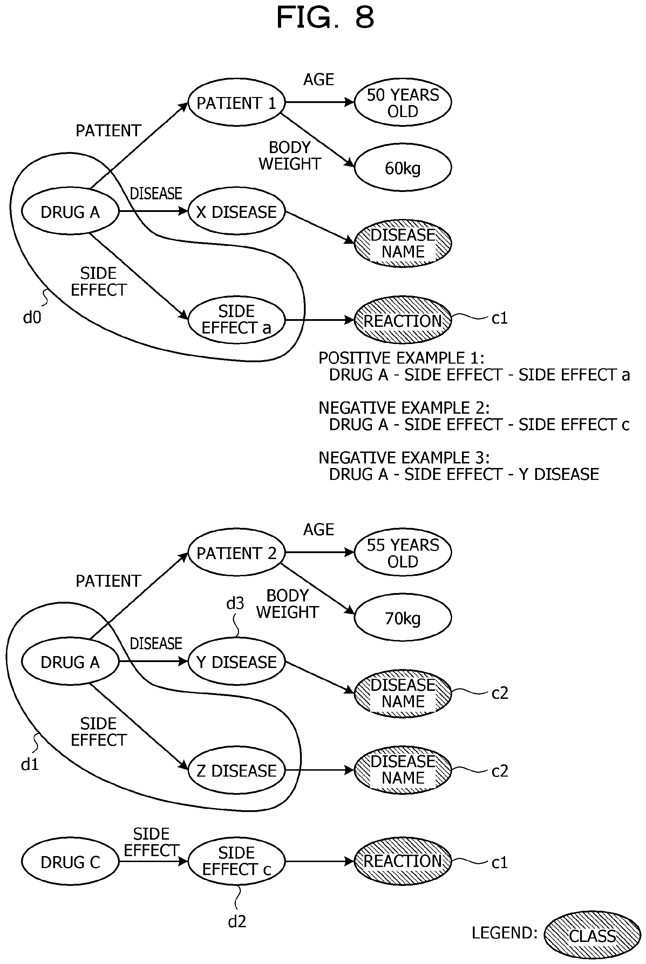

Generation of negative example datasets according to Embodiment 2 will be described with reference to FIG. 8. FIG. 8 is a diagram for explaining the generation of negative example datasets according to Embodiment 2. Knowledge graphs 22 are illustrated in FIG. 8. A data piece pointed from an object specifies a class. Classes are set in association with data pieces in the ontology 31.

The generation unit 12A acquires a graph dataset specifying a subject, a property, and an object from the knowledge graphs 22. The generation unit 12A sets the acquired graph dataset as a positive example graph dataset. In this example, the generation unit 12A acquires a graph dataset d0 specifying (drug A, side effect, side effect a) as relational information from the knowledge graphs 22. The generation unit 12A sets the acquired graph dataset d0 as a positive example graph dataset (see a positive example 1).

In a case where the class of every object is set in the ontology 31, the generation unit 12A generates a negative example graph dataset having another object in the same class as the class of the object contained in the positive example graph dataset by referring to the ontology 31. The class of the object "side effect a" contained in the positive example graph dataset d0 is set to be "reaction" marked with reference sign c1. Accordingly, the generation unit 12A generates a negative example graph dataset having another object "side effect c" d2 in the same class as the class "reaction" c1 of the object "side effect a" contained in the positive example graph dataset d0 (see a negative example 2).

The generation unit 12A acquires a graph dataset specifying a subject, a property, and an object from the knowledge graphs 22. The generation unit 12A sets the acquired graph dataset as a positive example graph dataset. In this example, the generation unit 12A acquires a graph dataset d1 specifying (drug A, side effect, Z disease) as relational information from the knowledge graphs 22. The generation unit 12A sets the acquired graph dataset d1 as a positive example graph dataset (not illustrated).

In a case where the class of every object is set in the ontology 31, the generation unit 12A generates a negative example graph dataset having another object in the same class as the class of the object contained in the positive example graph dataset by referring to the ontology 31. The class of the object "Z disease" contained in the positive example graph dataset d1 is set to be "disease name" marked with reference sign c2. Accordingly, the generation unit 12A generates a negative example graph dataset having another object "Y disease" d3 in the same class as the class "disease name" c2 of the object "Z disease" contained in the positive example graph dataset d1 (see a negative example 3).

In this way, the generation unit 12A is capable of excluding an object that is apparently unlinked, and stopping generation of unnecessary negative example graph datasets. As a result, the training unit 13 skips the training on unnecessary negative example graph datasets, so that it is possible to speed up convergence of the training and improve prediction accuracy.

### [Flowchart of Training Process]

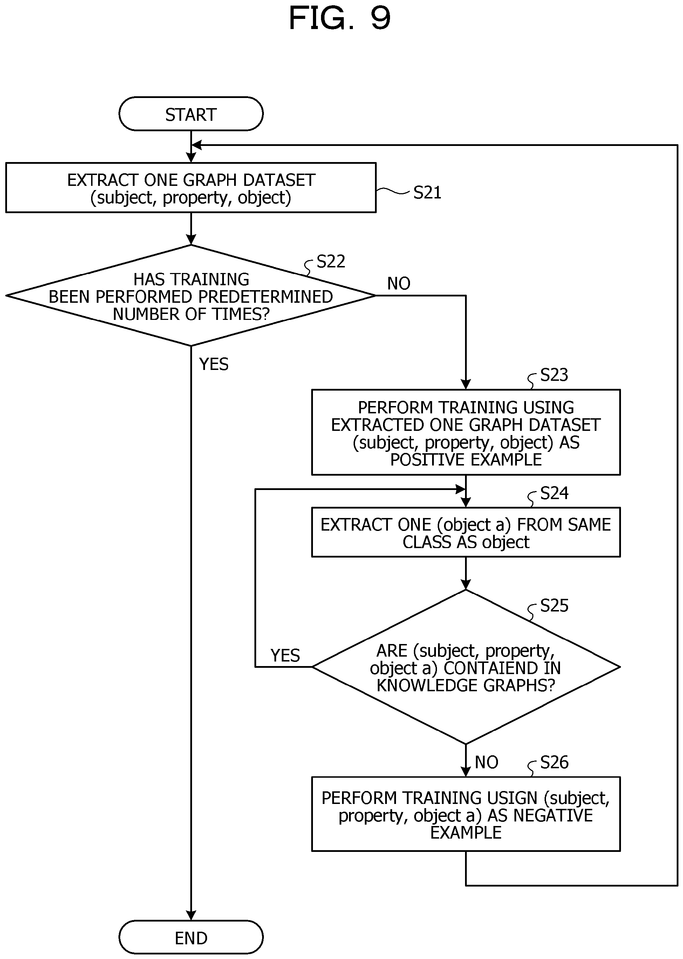

FIG. 9 is a view illustrating an example of a flowchart of a training process according to Embodiment 2. The conversion unit 11 converts the table data 21 into the knowledge graphs 22.

As illustrated in FIG. 9, the training unit 13 extracts one graph dataset (subject, property, object) from the knowledge graphs 22 (step S21). The training unit 13 determines whether or not training has been performed a predetermined number of times (step S22). When determining that the training has been performed the predetermined number of times (step S22; Yes), the training unit 13 ends the training process.

On the other hand, when determining that the training has not been performed the predetermined number of times (step S22; No), the training unit 13 performs the training using the extracted one graph dataset (subject, property, object) as a positive example (step S23). For example, the training unit 13 uses the positive example graph dataset to perform training for embedding in the knowledge graphs 22.

The generation unit 12A extracts one object a from the same class as the class of the object (step S24). For example, by referring to the ontology 31, the generation unit 12A generates negative example graph datasets each having an object in the same class as the class of the object contained in the positive example graph dataset.

The generation unit 12A determines whether or not (subject, property, object a) are contained in the knowledge graphs 22 (step S25). When determining that (subject, property, object a) are contained in the knowledge graphs 22 (step S25; Yes), the generation unit 12A proceeds to step S24 to extract a next object.

On the other hand, when determining that (subject, property, object a) are not contained in the knowledge graphs 22 (step S25; No), the training unit 13 performs the training on (subject, property, object a) as a negative example (step S26). For example, the training unit 13 uses the negative example graph dataset to perform the training for embedding in the knowledge graphs 22. After that, the training unit 13 proceeds to step S21 in order to extract a next graph dataset.

### [Effects of Embodiment 2]

According to Embodiment 2 described above, when the class of every object is set in the ontology 31, the information processing apparatus 1 further narrows down negative example graph datasets to be generated and generates only negative example graph datasets each having another object in the same class as the class of an object contained in a positive example graph dataset. In this way, the information processing apparatus 1 is capable of excluding an object that is apparently unlinked, and stopping generation of unnecessary negative example graph datasets. As a result, the information processing apparatus 1 skips the training on unnecessary negative example graph datasets, so that it is possible to speed up convergence of the training and improve prediction accuracy.

### [Embodiment 3]

Embodiment 2 has been described for the case where the information processing apparatus 1 narrows down negative example graph datasets to be generated and generates only negative example graph datasets each having an object in the same class as the class of an object contained in a positive example graph dataset when the class of every object is set in the ontology 31. However, the information processing apparatus 1 is not limited to this. Instead, in a case where a class of every object is not completely set in the ontology 31, the information processing apparatus 1 may use a class predictor and thereby predict the class of an object that is not set. The information processing apparatus 1 may generate a negative example graph dataset having an object in the same class as the class of the object contained in a positive example graph dataset. The reason why the ontology 31 is incomplete is that, for example, there is a case where a new disease is not yet defined in the international standards and is not set in the ontology 31.

Embodiment 3 will be described for a case where the information processing apparatus 1 predicts a class of an object that is not set by using a class predictor and generates negative example graph datasets.

### [Functional Configuration of Information Processing Apparatus]

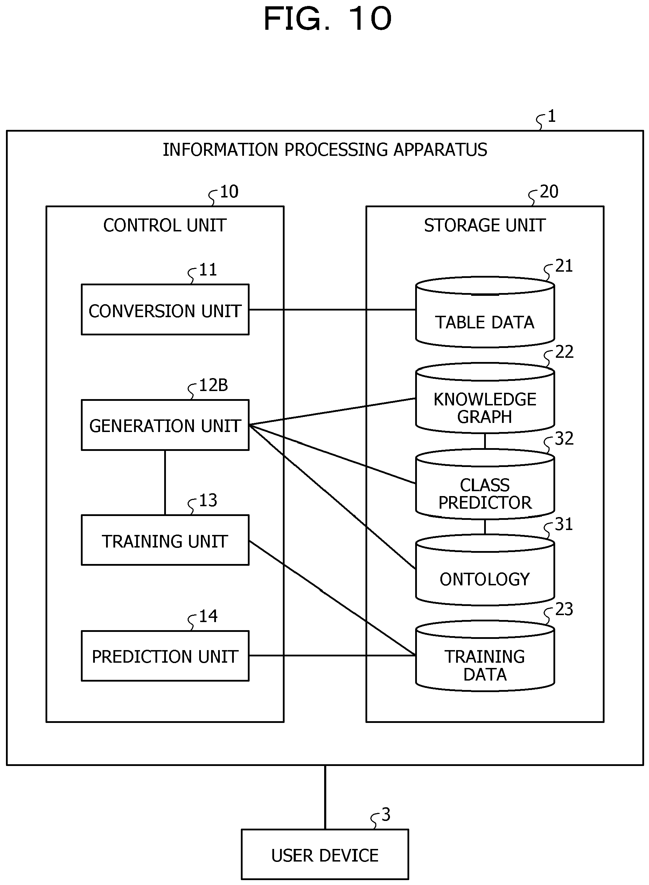

FIG. 10 is a functional block diagram illustrating a configuration of an information processing apparatus according to Embodiment 3. The same constituent elements as in the information processing apparatus 1 illustrated in FIG. 7 are assigned with the same reference sign, and the description of the identical constituent elements and operations thereof is omitted herein. Embodiment 3 is different from Embodiment 2 in that the generation unit 12A is replaced by a generation unit 12B. Embodiment 3 is different from Embodiment 2 in that a class predictor 32 is added.

Upon receipt of input of an object, the class predictor 32 predicts a class of the input object. For example, in a case where a class of the input object is found not to be set by referring to the ontology 31, the class predictor 32 analyzes a character string of the input object and searches for an object having a similar character string. If the class of the searched-out object is set, the class predictor 32 predicts the set class as the class of the input object. For example, when the ontology 31 is incomplete, the class predictor 32 uses a data piece whose class is set to predict a class of a data piece whose class is not set.

As an example, in a case where an input object is "... disease" or "... symptom", the class predictor 32 searches for an object having a character string ending with "disease" or "symptom". For example, when "disease name" is set as a class of the searched-out object, the class predictor 32 predicts that the set "disease name" class is the class of the input object. When the "reaction" class is set for the searched-out object, the class predictor 32 predicts that the set "reaction" class is the class of the input object.

The generation unit 12B generates positive and negative example graph datasets to be used for training. For example, the generation unit 12B acquires a graph dataset containing a subject, a property, and an object from the knowledge graphs 22. The generation unit 12B sets the acquired graph dataset as a positive example graph dataset. In a case where the class of every object is not completely set in the ontology 31, the generation unit 12B uses the class predictor 32 to predict the class of the object whose class is not set. By referring to the ontology 31 and the predicted class of the object, the generation unit 12B generates negative example graph datasets each having another object in the same class as the class of the object contained in the positive example graph dataset. For example, when objects in multiple classes may be linked to a property, the generation unit 12B generates negative example graph datasets by using the objects in the multiple classes as training targets.

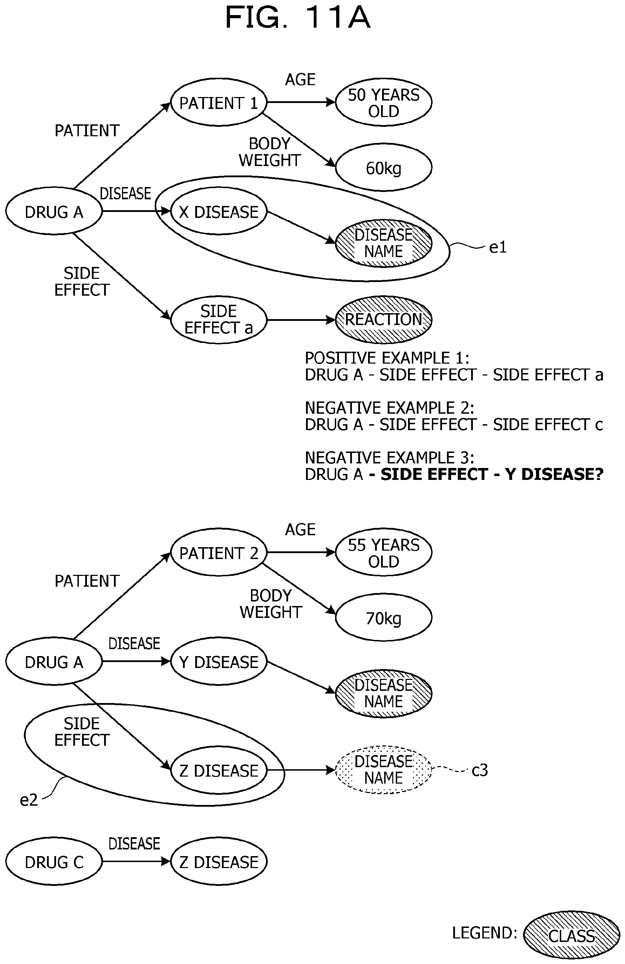

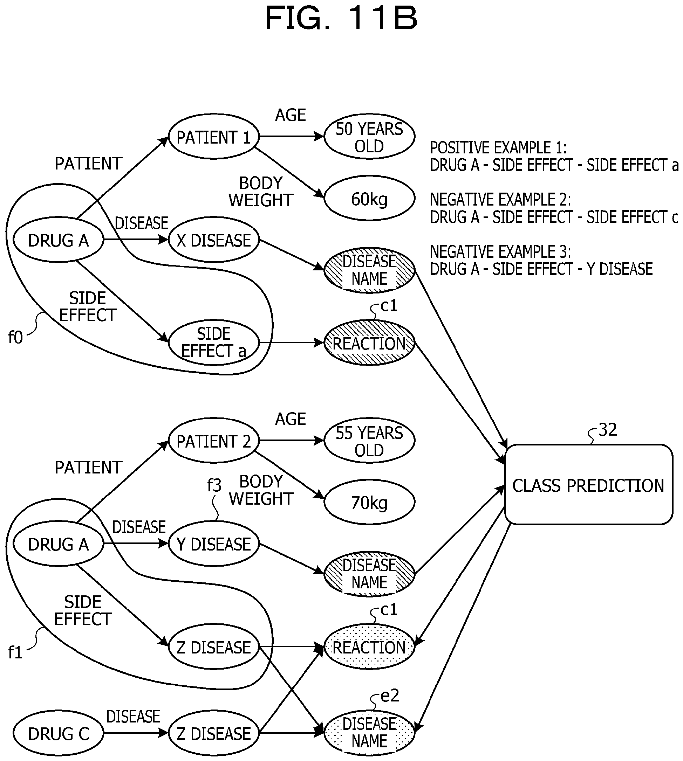

Generation of negative example datasets according to Embodiment 3 will be described with reference to FIGs. 11A and 11B. FIGs. 11A and 11B are diagrams for explaining the generation of negative example datasets according to Embodiment 3. Knowledge graphs 22 are illustrated in FIGs. 11A and 11B. A data piece pointed from an object specifies a class. Although classes are set in association with data pieces in the ontology 31, there is a data piece not associated with any class.

An overview of the generation of negative example datasets will be described with reference to FIG. 11A. As illustrated in FIG. 11A, since an object "X disease" in a "disease name" class is linked to a combination of (drug A, disease) (reference sign e1), it is possible to predict that "Z disease" may also be an object in the "disease name" class (reference sign c3). When "Z disease" is predicted to be in the "disease name" class, the generation unit 12B sets objects in the "reaction" class and objects in the "disease name" class as training targets for the combination of (drug A, side effect) because "Z disease" is linked to "side effect" (reference sign e2).

A specific example of the generation of negative example datasets will be described with reference to FIG. 11B. As illustrated in FIG. 11B, the generation unit 12B acquires a graph dataset specifying a subject, a property, and an object from the knowledge graphs 22. The generation unit 12B sets the acquired graph dataset as a positive example graph dataset. In this example, the generation unit 12B acquires a graph dataset f0 specifying (drug A, side effect, side effect a) as relational information from the knowledge graphs 22. The generation unit 12B sets the acquired graph dataset f0 as a positive example graph dataset (see a positive example 1).

In a case where the class of every object is not completely set in the ontology 31, the generation unit 12B uses the class predictor 32 to predict the class of the object whose class is not set. Since any class is not set for the object "Z disease", the generation unit 12B uses the class predictor 32 to predict that classes of the object "Z disease" may be "disease name" and "reaction". For example, the class predictor 32 predicts that "Z disease" may be in "disease name" and "reaction", and sets "disease name" and "reaction" as the classes. As an example, since "Z disease" is "... disease", the class predictor 32 searches for an object containing a character string ending with "disease" and searches out "Y disease". Because the "disease name" class is set for the searched-out object "Y disease", the class predictor 32 predicts that the "disease name" class may be the class of "Z disease". Additionally, since the property of "Z disease" is "side effect", the class predictor 32 predicts that "Z disease" may be in the "reaction" class.

By referring to the ontology 31 and the predicted class of the object, the generation unit 12B generates negative example graph datasets each having another object in the same class as the class of the object contained in the positive example graph dataset. The class of the object "side effect a" contained in the positive example graph dataset is set to be "reaction" marked with reference sign c1. Accordingly, the generation unit 12B generates a negative example graph dataset having another object "side effect c" (not illustrated) in the same class as the class "reaction" c1 of the object "side effect a" contained in the positive example graph dataset f0 (see a negative example 2).

The generation unit 12B acquires a graph dataset specifying a subject, a property, and an object from the knowledge graphs 22. The generation unit 12B sets the acquired graph dataset as a positive example graph dataset. In this example, the generation unit 12B acquires a graph dataset f1 specifying (drug A, side effect, Z disease) as relational information from the knowledge graphs 22. The generation unit 12B sets the acquired graph dataset f1 as a positive example graph dataset (not illustrated).

By referring to the ontology 31 and the predicted class of the object, the generation unit 12B generates negative example graph datasets each having another object in the same class as the class of the object contained in the positive example graph dataset. The classes of the object "Z disease" contained in the positive example graph dataset f1 are predicted to be "disease name" marked with reference sign e2 and "reaction" marked with reference sign c1. Accordingly, the generation unit 12B generates a negative example graph dataset having another object "Y disease" f3 in the same class as the class "disease name" e2 of the object "Z disease" contained in the positive example graph dataset f1 (see a negative example 3).

In this way, even when the ontology 31 is incomplete, the generation unit 12B is capable of stopping generation of unnecessary negative example graph datasets. As a result, the training unit 13 skips the training on unnecessary negative example graph datasets, so that it is possible to speed up convergence of the training and improve prediction accuracy.

### [Flowchart of Training Process]

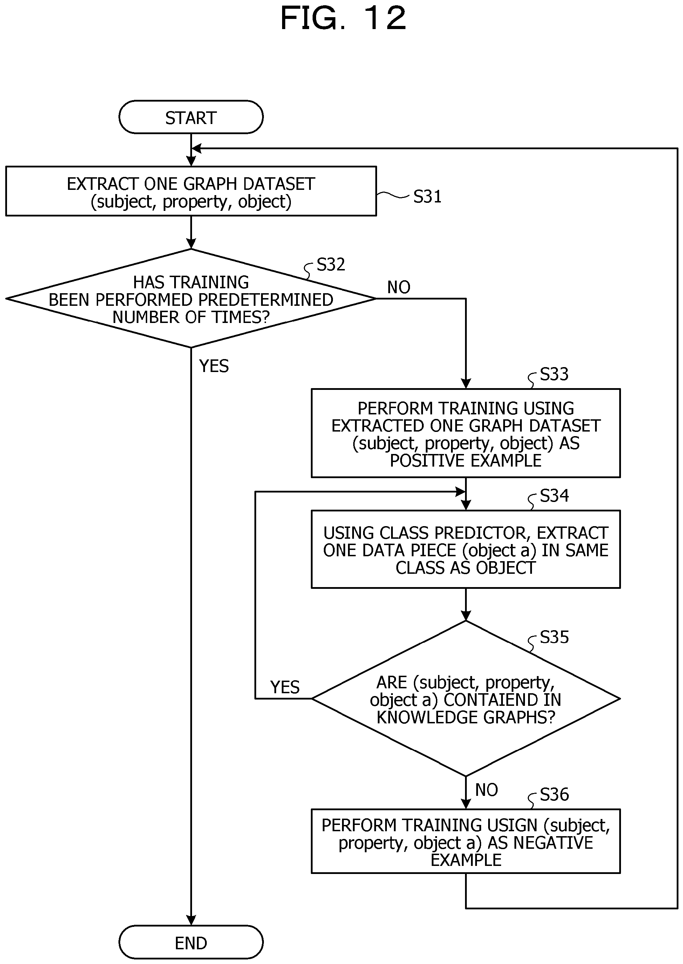

FIG. 12 is a diagram illustrating an example of a flowchart of a training process according to Embodiment 3. The conversion unit 11 converts the table data 21 into the knowledge graphs 22.

As illustrated in FIG. 12, the training unit 13 extracts one graph dataset (subject, property, object) from the knowledge graphs 22 (step S31). The training unit 13 determines whether or not training has been performed a predetermined number of times (step S32). When determining that the training has been performed the predetermined number of times (step S32; Yes), the training unit 13 ends the training process.

On the other hand, when determining that the training has not been performed the predetermined number of times (step S32; No), the training unit 13 performs the training using the extracted one graph dataset (subject, property, object) as a positive example (step S33). For example, the training unit 13 uses the positive example graph dataset to perform training for embedding in the knowledge graphs 22.

By using the class predictor 32, the generation unit 12B extracts one object a from the same class as the class of the object (step S34). For example, by referring to the ontology 31 and the predicted class of the object, the generation unit 12B generates a negative example graph dataset having another object in the same class as the class of the object contained in the positive example graph dataset.

The generation unit 12B determines whether or not (subject, property, object a) are contained in the knowledge graphs 22 (step S35). When determining that (subject, property, object a) are contained in the knowledge graphs 22 (step S35; Yes), the generation unit 12B proceeds to step S34 to extract a next object.

On the other hand, when determining that (subject, property, object a) are not contained in the knowledge graphs 22 (step S35; No), the training unit 13 performs the training on (subject, property, object a) as a negative example (step S36). For example, the training unit 13 uses the negative example graph dataset to perform the training for embedding in the knowledge graphs 22. After that, the training unit 13 proceeds to step S31 in order to extract a next graph dataset.

### [Effects of Embodiment 3]

According to Embodiment 3 described above, in a case where the class of every object is not completely set in the ontology 31, the information processing apparatus 1 uses the class predictor 32 to predict the class of an object that is not set. The information processing apparatus 1 generates a negative example graph dataset having another object in the same class as the class of the object contained in the positive example graph dataset. In this way, the information processing apparatus 1 is capable of stopping generation of unnecessary negative example datasets even when the ontology 31 is incomplete. As a result, the information processing apparatus 1 skips the training on unnecessary negative example graph datasets, so that it is possible to speed up convergence of the training and improve prediction accuracy.

The constituent elements of the information processing apparatus 1 illustrated do not necessarily have to be physically configured exactly as illustrated in the drawings. For example, the specific forms of distribution and integration of the information processing apparatus 1 are not limited to those illustrated in the drawings, and all or part thereof may be configured to be functionally or physically distributed or integrated in given units depending on various loads, usage states, and so on. For example, the generation unit 12 and the training unit 13 may be integrated with each other. The storage unit 20 may be coupled as an external device to the information processing apparatus 1 via a network.

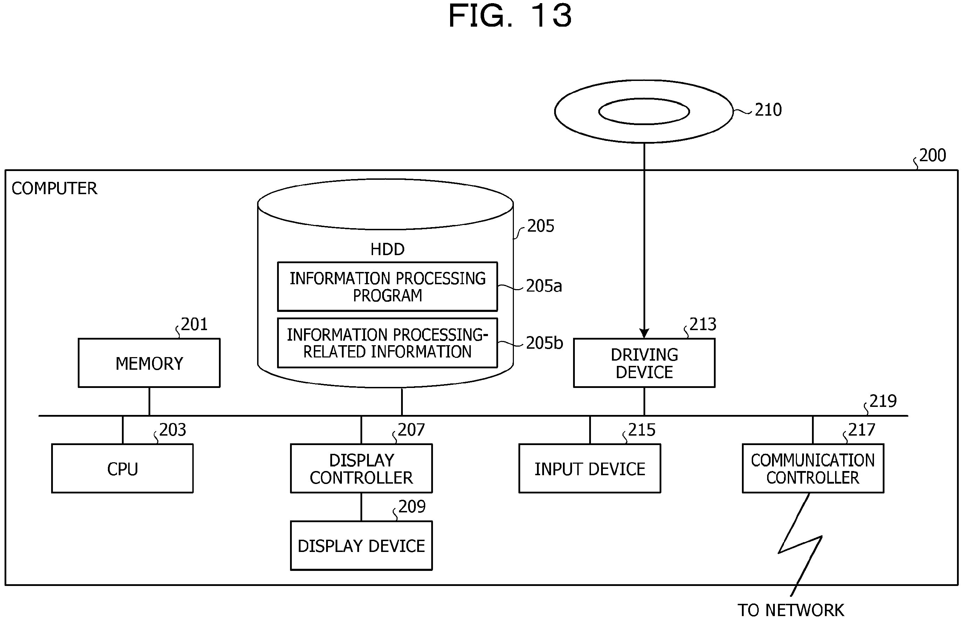

The various kinds of processing described in the above embodiments may be implemented by a computer such as a personal computer or a workstation executing a program prepared in advance. Hereinafter, an example of a computer that executes an information processing program implementing the same functions as those of the information processing apparatus 1 illustrated in FIG. 1 will be described. An information processing program for implementing the same functions as those of the information processing apparatus 1 will be described as an example. FIG. 13 is a diagram illustrating an example of a computer that executes the information processing program.

As illustrated in FIG. 13, a computer 200 includes a central processing unit (CPU) 203 that performs various kinds of arithmetic processing, an input device 215 that receives input of data from a user, and a display controller 207 that controls a display device 209. The computer 200 further includes a driving device 213 that reads a program and the like from a storage medium, and a communication controller 217 that exchanges data with another computer via a network. The computer 200 includes a memory 201 that temporarily stores various kinds of information and a hard disk drive (HDD) 205. The memory 201, the CPU 203, the HDD 205, the display controller 207, the driving device 213, the input device 215, and the communication controller 217 are coupled to each other via a bus 219.

The driving device 213 is, for example, a device for a removable disk 210. The HDD 205 stores an information processing program 205a and information processing-related information 205b.

The CPU 203 reads the information processing program 205a, develops the information processing program 205a on the memory 201, and executes the information processing program 205a as processes. Such processes correspond to the respective functional units of the information processing apparatus 1. The information processing-related information 205b corresponds to the table data 21, the knowledge graphs 22, and the training data 23. For example, the removable disk 210 stores various kinds of information such as the information processing program 205a.

The information processing program 205a may not necessarily have to be stored in the HDD 205 from the beginning. For example, the information processing program 205a may be stored in a "portable physical medium", such as a flexible disk (FD), a compact disk read-only memory (CD-ROM), a Digital Versatile Disk (DVD), a magneto-optical disk, or an integrated circuit (IC) card, to be inserted into the computer 200. The computer 200 may execute the information processing program 205a by reading the information processing program 205a from the portable physical medium.

## Claims

1. An information processing program that causes at least one computer to execute a process, the process comprising:
acquiring a graph dataset that includes one or more graphs each having a subject, a predicate, and an object from a knowledge graph;
setting the acquired graph dataset as a positive example graph dataset that includes one or more positive example graphs;
generating a negative example graph dataset that includes one or more negative example graphs each having a subject, a predicate, and an object, a predicate of each negative example graph being same as a predicate of one of the positive example graphs, an object of the negative example graph being different from an object of the one of the positive example graphs, the negative example graph being excluded from the negative example graph dataset when the object of the negative example graph is different from each object to which a predicate of any other of the positive example graphs is linked; and
training for embedding in the knowledge graph by using the positive example graph dataset and the negative example graph dataset.

2. The information processing program according to claim 1, wherein the generating includes generating the negative example graph dataset having an object in a class that is same as a class of the object of the graph dataset when the class of the object of the graph dataset and the class of the object of the negative example graph dataset is set in an ontology.

3. The information processing program according to claim 2, wherein the generating includes, when at least one class of selected from the object of the graph dataset and the class of the object of the negative example graph dataset is set out of the ontology, generating the negative example graph dataset having the object in a class that is same as a class of the object of the graph dataset by predicting a class of an object that is not set.

4. An information processing apparatus comprising:
an acquisition unit that acquires a graph dataset that includes one or more graphs each having a subject, a predicate, and an object from a knowledge graph;
a setting unit that sets the acquired graph dataset as a positive example graph dataset that includes one or more positive example graphs;
a generation unit that generates a negative example graph dataset that includes one or more negative example graphs each having a subject, a predicate, and an object, a predicate of each negative example graph being same as a predicate of one of the positive example graphs, an object of the negative example graph being different from an object of the one of the positive example graphs, the negative example graph being excluded from the negative example graph dataset when the object of the negative example graph is different from each object to which a predicate of any other of the positive example graphs is linked; and
a training unit that trains for embedding in the knowledge graph by using the positive example graph dataset and the negative example graph dataset.

5. The information processing apparatus according to claim 4, wherein the generation unit is further configured to
generate the negative example graph dataset having an object in a class that is same as a class of the object of the graph dataset when the class of the object of the graph dataset and the class of the object of the negative example graph dataset is set in an ontology.

6. The information processing apparatus according to claim 5, wherein the generation unit is further configured to
when at least one class of selected from the object of the graph dataset and the class of the object of the negative example graph dataset is set out of the ontology, generate the negative example graph dataset having the object in a class that is same as a class of the object of the graph dataset by predicting a class of an object that is not set.

7. An information processing method for a computer to execute a process comprising:
acquiring a graph dataset that includes one or more graphs each having a subject, a predicate, and an object from a knowledge graph;
setting the acquired graph dataset as a positive example graph dataset that includes one or more positive example graphs;
generating a negative example graph dataset that includes one or more negative example graphs each having a subject, a predicate, and an object, a predicate of each negative example graph being same as a predicate of one of the positive example graphs, an object of the negative example graph being different from an object of the one of the positive example graphs, the negative example graph being excluded from the negative example graph dataset when the object of the negative example graph is different from each object to which a predicate of any other of the positive example graphs is linked; and
training for embedding in the knowledge graph by using the positive example graph dataset and the negative example graph dataset.

8. The information processing method according to claim 7, wherein the generating includes generating the negative example graph dataset having an object in a class that is same as a class of the object of the graph dataset when the class of the object of the graph dataset and the class of the object of the negative example graph dataset is set in an ontology.

9. The information processing method according to claim 8, wherein
the generating includes, when at least one class of selected from the object of the graph dataset and the class of the object of the negative example graph dataset is set out of the ontology, generating the negative example graph dataset having the object in a class that is same as a class of the object of the graph dataset by predicting a class of an object that is not set.
